# FASCICULE DE BREVET EUROPEEN

(11) **EP 2 922 603 B1**
(45) Date de publication et mention de la délivrance du brevet: **13.12.2017**
(21) Numéro de dépôt: 13805430.9
(22) Date de dépôt: 17.10.2013
(51) Int. Cl.: B01D 1/28, B01D 3/00, C07C 15/08, C10G 7/00

(54) **METHODE DE RECUPERATION DE CHALEUR A BASSE TEMPERATURE ET APPLICATION DE LA METHODE AU COMPLEXE AROMATIQUE**
VERFAHREN ZUR RÜCKGEWINNUNG VON NIEDERTEMPERATURWÄRME UND ANWENDUNG DES VERFAHRENS BEI EINEM AROMATISCHEN KOMPLEX
METHOD FOR RECOVERING LOW-TEMPERATURE HEAT AND APPLICATION OF SAID METHOD TO AN AROMATIC COMPLEX

(30) Priorité: 22.11.2012 FR 1203145
(43) Date de publication de la demande: 30.09.2015
(73) Titulaire: AXENS, 92508 Rueil Malmaison Cedex (FR)
(72) Inventeur: DUNET, Alain, 91300 Massy (FR); PREVOST, Isabelle, 92500 Rueil Malmaison (FR); LEFLOUR, Thierry, 75015 Paris (FR)
(74) Mandataire: Schmitt, Nicolas A.J.
(86) Numéro de dépôt international: PCT/FR2013/052481
(87) Numéro de publication internationale: WO 2014/080102

(56) Documents cités:
- EP-A2- 0 401 631
- DE-A1- 19 903 781
- GB-A- 776 154
- US-A- 4 566 947
- US-A- 4 783 242
- US-A1- 2012 048 714
- US-B1- 6 589 395

## Description

### DOMAINE DE L'INVENTION

L'invention se situe dans le domaine des procédés de raffinage et de pétrochimie faisant appel à au moins une colonne de fractionnement et générant de la vapeur basse pression.

L'invention s'applique en particulier au cas d'un complexe aromatiques qui fait appel à un grand nombre de colonnes à distiller, Le principe de l'invention réside dans la génération de vapeur basse pression au niveau de certaines colonnes, la vapeur BP ainsi générée étant utilisée comme fluide caloporteur, avec ou sans compression intermédiaire, par exemple pour le rebouillage d'autres colonnes ayant subi éventuellement un abaissement de pression qui permet au rebouilleur desdites colonnes de fonctionner à plus basse température.

La vapeur BP générée peut également, avec ou sans compression intermédiaire, être utilisée pour préchauffer d'autres fluides dont la température est inférieure à celle de la vapeur générée.

De manière plus générale, on modifie les conditions opératoires et/ou la configuration du procédé de façon à réduire la part de sa consommation de chaleur à haut niveau thermique au profit de la part de sa consommation de chaleur à bas niveau thermique (cette chaleur à bas niveau thermique pouvant être produite dans le complexe),

L'utilisation de la vapeur BP ainsi générée, combinée avec la modification des conditions opératoires de certaines colonnes permet, pour un complexe aromatique, un gain énergétique global substantiel sur la consommation de combustible et d'électricité,

Ces gains sont tout à fait significatifs dans le contexte de réduction des couts énergétiques et peuvent s'appliquer à différents procédés en particulier aux complexes aromatiques.

La présente invention définit les moyens permettant de récupérer la chaleur considérée comme perdue en raison de son faible niveau de température (autour de 100 à 180°C) en générant de la vapeur basse pression qui peut éventuellement, selon les utilisations, être recomprimée pour être utilisée comme fluide caloporteur dans les rebouilleurs de certaines colonnes et/ou les préchauffeurs de certains fluides procédés.

### EXAMEN DE L'ART ANTERIEUR

L'amélioration de l'efficacité énergétique des procédés est une tendance de fond dans le contexte économique actuel. Jusqu'à une date récente la chaleur disponible dans certaines échangeurs, et notamment les aeroréfrigérants, à une température située dans l'intervalle de 100°C à 180°C était considérée comme perdue. En effet, une chaleur à ce niveau de température ne trouvait pas d'utilisation par échange direct avec un fluide procédé "froid" issu d'un procédé de raffinage ou de pétrochimie, par exemple un complexe aromatique.

Il est connu de l'homme du métier d'augmenter le niveau de température de ces quantités de chaleur de plus en plus importantes en modifiant des conditions opératoires du procédé et/ ou en complexifiant le schéma de procédé lui même.

Par exemple, au niveau d'une unité de fractionnement, telle qu'une colonne à distiller, l'homme du métier sait pratiquer une augmentation du niveau de pression de la colonne à distiller, les vapeurs de tête étant alors obtenues à plus haute température. Mais cette augmentation de pression opératoire s'accompagne inévitablement d'une augmentation de la quantité de chaleur nécessaire au rebouilleur, lui même fonctionnant à température plus élevée.

Le document US 4,566,947 décrit un procédé de production de xylènes à partir d'une charge comprenant des aromatiques en C8, C9 et des composés plus lourds. Le procédé décrit dans D1 comprend a) une étape de distillation, b) une étape de séparation des xylènes par adsorption et c) une étape de récupération du désorbant utilisé dans l'étape d'adsorption.

Le document US 2012/0048714 décrit un complexe aromatique dans lequel un transfert de chaleur est organisé entre deux colonnes dudit complexe par condensation du flux de tête de l'une des colonnes vers le rebouilleur de l'autre colonne, notamment entre la première colonne des xylènes et le rebouilleur de la colonne d'extrait.

### DESCRIPTION SOMMAIRE DES FIGURES

La figure 1a est un schéma selon l'art antérieur qui montre la configuration de base d'une colonne à distiller équipée de son condenseur de tête et de son rebouilleur en fond, ledit rebouilleur étant un four.
La figure 1b selon l'invention montre comment il est possible de réduire la pression opératoire d'une colonne à distiller par un système de compression et de refroidissement des vapeurs de tête de ladite colonne. Cette modification de pression opératoire permet d'abaisser la température du fluide chaud requis pour rebouillir cette colonne, ainsi que la quantité de chaleur requise pour effectuer ce rebouillage.
La figure 2 selon l'invention illustre la génération de vapeur basse pression et moyenne pression à partir du condenseur de tête de la colonne notée C4, et l'utilisation de vapeur moyenne pression comme fluide caloporteur au rebouilleur de la colonne notée C7.
La figure 3 est un schéma de procédé d'un complexe aromatique selon l'art antérieur.
La figure 4 est un schéma de procédé d'un complexe aromatique selon l'invention, c'est à dire incluant plusieurs circuits de vapeur basse pression (marqué en traits gras) qui concernent un certain nombre de colonnes.

### DESCRIPTION SOMMAIRE DE L'INVENTION

La présente invention peut se définir comme une méthode de récupération de la chaleur basse température contenue dans certains échangeurs de chaleur d'un procédé de raffinage ou de pétrochimie appelé par la suite "complexe".

La méthode objet de l'invention consiste essentiellement à créer un réseau de vapeur basse pression (BP) générée à partir de certaines source de chaleurs, à recomprimer (totalement ou partiellement) cette vapeur basse pression pour la transformer en vapeur moyenne pression (MP), et à utiliser les vapeurs (BP et MP) ainsi générées comme source de chaleur pour différents services du procédé. En particulier, les vapeurs BP et MP générées peuvent être utilisées pour apporter les calories nécessaires au rebouilleur d'un certain nombre de colonnes à distiller, après avoir éventuellement abaissé la pression de fonctionnement desdites colonnes, de manière à diminuer la température opératoire de leurs rebouilleurs.

Dans le contexte de la présente invention, on appelle vapeur basse pression (BP) une vapeur de pression allant de 1 à 7 bars absolus. On appelle vapeur moyenne pression (MP) une vapeur allant de 7 bars absolus à une valeur comprise entre 15 et 17 bars absolus, et on appelle vapeur haute pression (HP) une vapeur de pression supérieure à 17 bars absolus.

Plus précisément, la méthode selon la présente invention peut se définir comme une méthode de récupération de chaleur à basse température, c'est à dire comprise entre 100°C et 180°C, dans un procédé de raffinage ou de pétrochimie, appelé "complexe" comportant au moins une colonne à distiller, ladite méthode consistant dans les étapes suivantes :
1) Génération de la vapeur basse pression (c'est à dire à une pression comprise entre 1 bar abs et 7 bar absolu (abs) (1bar=0,1MPa) par échange avec au moins une source de chaleur à basse température,
2) Augmentation de la pression de la vapeur générée dans au moins une étape de compression, pour créer de la vapeur MP,
3) Modification des conditions opératoires et/ou de la configuration du procédé de façon à réduire la part de la consommation de chaleur à haut niveau thermique au profit de la part de consommation de chaleur à bas niveau thermique,
4) Utilisation d'au moins une partie de la vapeur BP issue de l'étape 1 et de la vapeur MP issue de l'étape 2 pour apporter les calories nécessaires aux postes de consommation de chaleur basse température issus de l'étape 3.

Selon une variante de la présente invention, on utilise l'autre partie des vapeurs issues de l'étape 1 pour générer de l'électricité.

Selon une variante de la présente invention, l'étape 2 est réalisée au moyen d'un compresseur à deux étages.

Selon une autre variante de la présente invention, l'étape 2 est réalisée au moyen d'un éjecteur utilisant de la vapeur HP comme fluide moteur.

L'étape 3 peut se réaliser de plusieurs façons non exclusives entre elles, c'est à dire que toute combinaison des différentes manières de réaliser l'étape 3 reste dans le cadre de la présente invention.
- Dans un première mode de réalisation, l'étape 3 est réalisée au moyen d'une diminution de la pression opératoire d'une ou plusieurs colonnes de distillation du procédé de manière à abaisser la température de leur rebouilleur au dessous de la température de la vapeur issue de l'étape 2.
- Dans un second mode de réalisation, l'étape 3 est réalisée au moyen d'une modification du réseau d'échangeur de préchauffage des charges des colonnes de distillation du complexe. Selon la méthode de la présente invention, une partie au moins des sources de chaleurs basse température permettant de générer de la vapeur basse pression (BP) est choisie parmi les condenseurs de certaines colonnes à distiller du complexe formant un premier ensemble E1.

Selon une variante de la présente invention, la vapeur basse pression issue de l'étape 1 est utilisée directement comme fluide caloporteur apporté aux rebouilleurs de certaines colonnes du complexe.

Selon une autre variante de la présente invention, la vapeur basse pression issue de l'étape 1 est utilisée après recompression comme fluide caloporteur apporté aux rebouilleurs de certaines colonnes du complexe.

Les colonnes dont les rebouilleurs utilisent directement ou après recompression la vapeur basse pression issue de l'étape 1 forment un second ensemble E2, distinct de E1. Ensemble distinct signifie dans le contexte de la présente invention que E1 et E2 n'ont pas de colonne en commun.

Selon une autre variante, compatible avec les deux précédentes, la vapeur basse pression issue de l'étape 1 ou de l'étape 2 est utilisée comme fluide caloporteur pour apporter de la chaleur à des fluides procédés dans des échangeurs de chaleur.

Selon une autre variante, toujours compatible avec les précédentes, la vapeur basse pression issue de l'étape 1 ou de l'étape 2 est utilisée comme fluide caloporteur pour apporter de la chaleur à l'air de combustion des fours dans des préchauffeurs d'air.

Enfin selon une variante de la méthode de la présente invention, certaines colonnes de distillation de l'ensemble E2 fonctionnent à pression opératoire plus faible afin de réduire le niveau de température requis pour leur rebouillage.

La présente invention peut s'appliquer à tout type de procédés possédant des sources de chaleur à basse température susceptibles de générer de la vapeur basse pression.

En particulier, la présente invention s'applique au procédé dit "complexe aromatique" faisant appel à au moins une unité de distillation extractive (P1), une unité d'adsorption du paraxylène (P2), une unité d'isomérisation des xylènes (P3) et une unité de transalkylation (P4), les effluents desdites unités étant séparés dans des colonnes à distiller notées de C1 à C11 avec la signification suivante :
C1 colonne de reformat, C2 colonne des xylènes, C3 colonne des aromatiques lourds, C4 colonne de raffinat, C5 colonne d'extrait, C6 colonne de purification, C7 deheptaniseur, C8 stripeur, C9 colonne de benzène, C10 colonne de toluène, C11 colonne de stabilisation, complexe aromatique dans lequel :
   - les source de chaleurs basse température qui sont choisies dans l'ensemble E1 formé par les colonnes à distiller suivantes C3, C4 et C5 qui permettent de générer de la vapeur basse pression (BP)
   - les colonnes dont on abaisse la pression, et auxquelles on apporte les calories sous forme de vapeur moyenne pression (MP) après compression de la vapeur BP générée par l'ensemble E1 (ou un quelconque de ses sous ensembles), qui sont choisies dans l'ensemble formé par les colonnes suivantes C7, C8, C10, C11,
   - les colonnes auxquelles on apporte les calories de la vapeur basse pression générée par l'ensemble E1 (ou un quelconque de ses sous ensembles), sans abaissement de pression. Dans le cas d'application au complexe aromatique, il s'agit de la colonne C9.

Dans le cas de l'application au complexe aromatique, l'ensemble E2 des colonnes utilisant la vapeur générée par les colonnes E1 est donc constitué des colonnes C7, C8, C9, C10 et C11.

### DESCRIPTION DETAILLEE DE L'INVENTION

Dans la description qui suit on parle de "procédé" ou "complexe" pour désigner tout procédé de raffinage ou de pétrochimie comportant au moins deux colonnes à distiller.

Cette définition est très large et comprend, par exemple, le procédé de craquage catalytique des essences, et le procédé de production de paraxylène ou de métaxylènes à partir de coupes aromatiques dit "complexe aromatique",

La description qui suit et l'exemple qui illustre la présente invention sont donnés dans le cas du complexe aromatiques, mais il est bien entendu que celui ci ne constitue qu'un cas d'application et ne limite en aucun cas la portée de la méthode exposée.

Le procédé selon la présente invention peut se définir, de manière générale, comme une méthode de récupération de chaleur à basse température pour produire de la vapeur basse pression qui, après recompression, peut être utilisée pour différents services, dont le chauffage des rebouilleurs de certaines colonnes du procédé considéré, éventuellement après un abaissement de la pression de fonctionnement desdites colonnes, de façon à permettre au rebouilleur desdites colonnes de fonctionner à plus basse température.

La méthode de récupération de chaleur à basse température selon la présente invention consiste essentiellement à générer de la vapeur à partir de la chaleur à basse température disponible dans le complexe aromatique.

En pratique, ces sources de chaleur sont constituées par les aérocondenseurs des vapeurs de tête de certaines colonnes, et la génération de vapeur BP est obtenue en les remplaçants par des échangeurs appelés vaporiseur ou générateur de vapeur BP.

La vapeur ainsi générée se trouve donc à un niveau de température typiquement compris entre 100°C et 170°C.

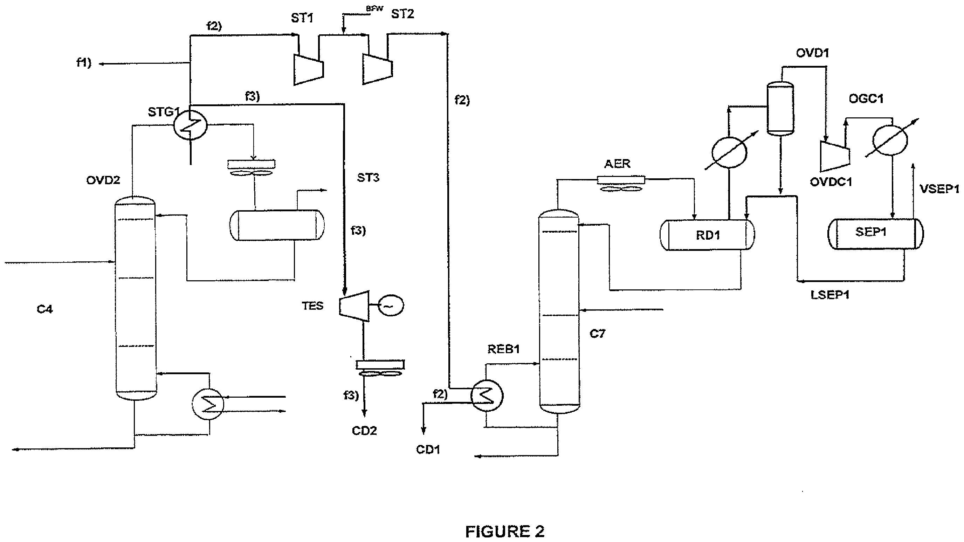

Cette vapeur BP à température comprise entre 100°C et 170°C peut être utilisée de différentes manières :
a) Soit cette vapeur peut être envoyée directement comme fluide caloporteur pour être utilisé en différents points du complexe aromatiques, en particulier (et de façon non limitative) aux rebouilleurs de certaines colonnes. Cette utilisation correspond au circuit du flux f1 de la figure 2.
b) Soit elle peut être envoyée dans un circuit de compression comprenant un ou plusieurs étages de compression générant de la vapeur moyenne pression (MP) permettant de produire un ou plusieurs fluides surchauffés. Ces fluides surchauffés sont ensuite condensés en cédant leur chaleur de condensation en différents points du complexe aromatiques, notamment aux rebouilleurs de certaines colonnes dont la pression opératoire a éventuellement été diminuée. Pour obtenir un échange de chaleur suffisamment efficace, on admet que la température des fluides chauds doit préférentiellement se situer entre 5°C et 15°C, au dessus de la température des zones où s'effectue le transfert de chaleur. Cette utilisation correspond au circuit du flux f3 de la figure 2.
c) Soit cette vapeur BP peut être introduite dans un expandeur de manière à produire de l'électricité. On utilise alors préférentiellement des turbo expandeur permettant une condensation de la vapeur au dessous de la pression atmosphérique (dit en abrégé "turbo expandeur sous vide"). Cette utilisation correspond au circuit du flux f2 de la figure 2.

Les vapeurs BP résultant des utilisations a), b) ou c) se retrouvent à l'état condensé, et le fluide résultant peut être traité dans un réseau de condensats de manière à constituer un fluide apte à être recyclé à l'entrée des différents vaporiseurs de manière à constituer une boucle eau/vapeur fermée (avec un appoint d'eau déminéralisée).

On construit ainsi un circuit fermé d'utilités basé
- sur la génération de vapeur basse pression au niveau du condenseur d'une première série de colonnes à distiller,
- sur l'utilisation de cette vapeur (comprimée ou pas), comme agent caloporteur au niveau de certains fluides froids du procédé, en particulier les rebouilleurs d'une seconde série de colonnes à distiller, ou comme fluide moteur pour générer de l'électricité puis,
- sur la récupération des condensats après traitement de manière à reconstituer le fluide destiné aux vaporisations.

### DESCRIPTION DETAILLEE DES FIGURES

La figure la montre une colonne (COL1) selon l'art antérieur munie d'un ballon de reflux (CD1) faisant appel à un aeroréfrigérant (AER) qui refroidit le flux de tête de la colonne et d'un rebouilleur qui est un four (F1).

La pression de cette colonne est minimisée de façon à pouvoir condenser les vapeurs à une température au moins supérieure à celle des utilités froides disponibles, tout en limitant les pertes de produit via le fluide dégazé en tête de colonne. Pour la stabilisation d'effluents de la section réactionnelle contenant des mélanges de corps ayant des volatilités très différentes, cela conduit à opérer les colonnes sous une pression de l'ordre de 5 à 12 bars absolus.

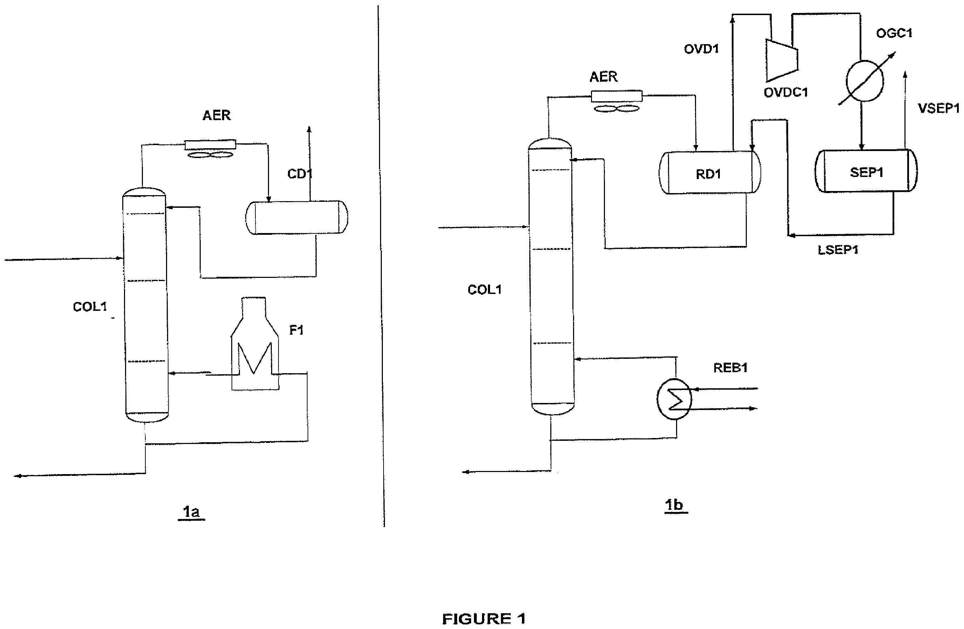

La figure 1b montre les modifications à faire subir en tête de colonne (COL1) pour compenser l'abaissement de pression opératoire de la dite colonne, et récupérer le supplément de phase gazeuse occasionné par ledit abaissement de pression.

En fond de colonne, le four a été remplacé par un échangeur de chaleur fonctionnant à plus basse température,

De manière plus précise, le système de condensation des vapeurs de tête de la colonne (COL 1) modifié selon la présente invention peut se décrire de la façon suivante :
Les vapeurs de tête de la colonne (COL 1) sont partiellement condensées, par exemple dans un aéroréfrigérant (AER) et récupérées sous forme de phase mixte dans le ballon de reflux (RD1) de la colonne. Le gaz OVD1 qui s'échappe du ballon de reflux (RD1) est envoyé à l'aspiration du compresseur de gaz de tête (OVDC1).

Le gaz au refoulement du compresseur (OVDC1) est partiellement (ou totalement) condensé via le condenseur (OGC1). Le flux en sortie du condenseur (OGC1) est récupéré et séparé dans le ballon (SEP1).

Le liquide (LSEP 1) du ballon (SEP1) est renvoyé au ballon de reflux (RD1) et le gaz VSEP1 issu du ballon (SEP1) est envoyé dans une autre partie de l'unité ou en limite d'unité.

La figure 2 illustre les utilisations correspondant aux circuits des flux f2) et f3) de la vapeur BP issue de la condensation des vapeurs de tête d'une colonne.

La chaleur de condensation des vapeurs de tête (OVD2) de la colonne C4 permet à travers l'échangeur de chaleur (STG1) de vaporiser un fluide liquide. La pression de ce liquide est choisie pour que sa température de vaporisation soit inférieure à celle des vapeurs OVD2 à condenser.

La vapeur basse pression ainsi obtenue se divise en 3 flux f1), f2) et f3) qui empruntent vdesz circuits différents que l'on peut décrire de la manière suivante :
- flux f1) : une partie de la vapeur BP peut être envoyée directement comme fluide caloporteur au rebouilleur de certaines colonnes. Cette utilisation de vapeur BP très usuelle pour l'homme du métier, n'est pas illustrée sur la figure 2.
- flux f2) : une autre partie de la vapeur BP est envoyée vers deux étages de compression (ST1 et ST2) de manière à générer de la vapeur moyenne pression qui est utilisée comme fluide caloporteur au rebouilleur (REB1) de la colonne C7. En aval du rebouilleur (REB1) la vapeur est condensée (CD1). Cette utilisation est selon la présente invention.
- flux f3) : une autre partie de la vapeur BP est introduite dans une turbine à vapeur BP (TES) de manière à produire de l'électricité. Au refoulement de la turbine à vapeur (TES), le fluide est totalement condensé dans un aeroréfrigérant pour produire un condensat (CD2).

Cette utilisation est également selon l'invention.

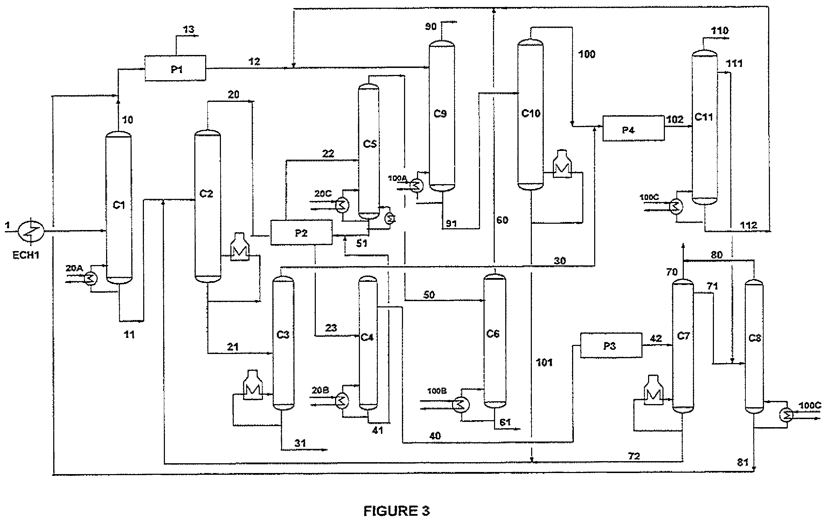

La figure 3 montre un schéma de procédé typique d'un complexe aromatique selon l'art antérieur, et est décrite en détail dans le paragraphe suivant.

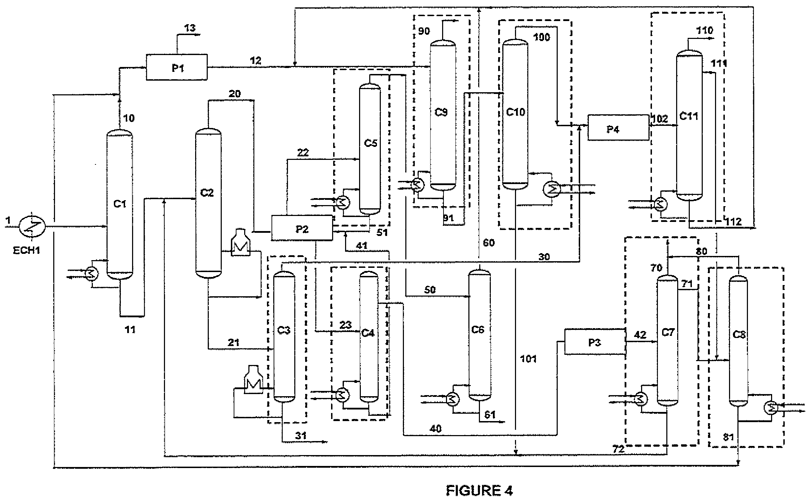

La figure 4 fait ressortir les zones du complexe aromatique qui sont modifiées selon la présente invention par rapport au procédé typique de l'art antérieur (figure 3) en entourant lesdites zones d'un trait pointillé.

Dans les figures 2 et 4, par soucis de simplification, les sections de reflux, ballons de reflux ou condenseurs ne sont pas représentés.

### EXEMPLE :

L'exemple qui suit est une application de la méthode précédemment décrite au cas d'un complexe aromatiques. La bonne compréhension de cette application nécessite une description du complexe aromatique lui-même qui est faite au moyen de la figure 3 selon l'art antérieur.

La figure 4 selon l'invention montre les zones du complexe aromatique qui ont été modifiées selon les moyens de la présente invention :
- réduction de la pression opératoire de certaines colonnes du complexe,
- utilisation des effluents de tête de certaines colonnes pour la génération de vapeur,
- utilisation de la vapeur produite à l'intérieur du complexe aromatique

Un complexe aromatique tel que décrit dans l'exemple qui suit, peut se définir comme une série d'étapes de conversion et de séparation destinée à produire des composés aromatiques à huit atomes de carbone, appelé xylènes, et plus particulièrement le paraxylène noté PX, à partir d'une charge riche en composés aromatiques allant du benzène à des composés aromatiques à plus de 10 atomes de carbone (notés C10+) provenant d'une unité de reformage catalytique.

Elle doit présenter des teneurs en composés soufrés, azotés et oléfines très faible à nulle, car ces composés peuvent affecter les performances et la durée de vie de certains catalyseurs et des tamis moléculaires mis en oeuvre dans les unités du complexe.

### Colonne de reformat (C1)

La charge à traiter est envoyée via la ligne (1) et l'échangeur ECH1 à une première colonne à distiller notée (C1) qui sépare le toluène et les composés plus légers des composés plus lourds (allant de C8 à C10+).

Voir la table 1 ci dessous pour la configuration du rebouilleur et du condenseur de cette colonne selon l'art antérieur.

### Unité de distillation extractive (P1)

Le toluène et les composés plus légers récupérés en tête de la colonne C1 sont envoyés via la ligne 10 à une unité de distillation extractive notée (P1) qui sépare une coupe C6-C7 purement aromatique d'un produit appelé « raffinat » constitué de composés paraffiniques.

Ledit raffinat est envoyé à l'extérieur du complexe via la ligne (13). Le solvant utilisé préférentiellement dans cette unité de distillation extractive est la N-formylmorpholine (NFM).

### Colonne des xylènes (C2)

Les composés aromatiques C8-C10+ récupérés au fond de la colonne (C1) sont envoyés via la ligne (11) à la colonne (C2) dite colonne des xylènes qui sépare les composés en C9 et plus lourds des composés aromatiques en C8 (appelés xylènes) qui alimentent les unités du complexe situées en aval.

Selon l'art antérieur, la chaleur récupérée par la condensation des vapeurs de tête de la colonne C2 est utilisée pour apporter la chaleur nécessaire aux rebouilleurs de la colonne C1, de la colonne C4 (dite colonne de raffinat) et de la colonne C5 (dite colonne d'extrait) qui seront examinées plus loin.

Dans ce but, la pression opératoire en tête de la colonne C2 est généralement maintenue à la pression minimale requise (généralement comprise entre 7,0 et 9,0 bars absolus), qui permet une température de condensation des vapeurs de tête de la colonne de xylènes (C2) suffisante pour être utilisée comme fluide caloporteur pour les rebouilleurs des colonnes (C1), (C4) et (C5).

Voir la table 1 ci dessous pour la configuration du rebouilleur et du condenseur de cette colonne selon l'art antérieur.

### Unité d'adsorption du paraxylène (P2)

La coupe xylènes, c'est à dire une coupe de C8 aromatiques contenant du paraxylène, du méta xylène de l'ortho xylène et de l'éthylbenzène, est donc récupérée en tête de la colonne C2 et est envoyée via la ligne (20) à l'unité d'adsorption P2 qui récupère sélectivement le para xylène contenu dans ladite coupe. L'unité d'adsorption P2 produit donc un mélange de paraxylène et de désorbant (appelé l'extrait) et un mélange des autres composés C8-aromatiques et de désorbant (appelé le raffinant).

L'adsorbant est un tamis moléculaire spécialement dédié à l'adsorption du paraxylène, c'est à dire qu'il présente une affinité particulièrement élevée envers ce composé.

Un solide adsorbant couramment utilisé est une zéolithe de type faujasite mise en forme avec un liant silicique échangée au baryum ou au potassium. Le désorbant préférentiellement utilisé est le paradiéthylbenzène (PDEB).

### Colonne d'extrait (C5)

Le flux d'extrait issu de la colonne d'adsorption et contenant le paraxylène et du désorbant est envoyé via la ligne (22) à la colonne d'extrait (C5) qui sépare le paraxylène du désorbant.

Le désorbant récupéré au fond de la colonne (C5) est renvoyé à la colonne d'adsorption via la ligne 51.

La colonne d'extrait est préférentiellement opérée à basse pression, c'est à dire dans une gamme de pression allant de 1,0 à 2,0 bars absolus au ballon de reflux, de manière à minimiser à la fois la température du rebouilleur et la quantité de chaleur à fournir audit rebouilleur.

Selon l'art antérieur, la condensation des vapeurs de tête de la colonne d'extrait (C5) est réalisée au moyen d'un aeroréfrigérant.

Voir table 1 pour la configuration du rebouilleur et du condenseur de cette colonne selon l'art antérieur.

### Colonne de Purification (C6)

Le flux de tête de la colonne (C5) est envoyé via la ligne (50) à la colonne de purification (C6) qui sépare le toluène (qui a été partiellement extrait avec le paraxylène) du paraxylène. Le toluène qui sort en tête de la colonne (C6) est envoyé via la ligne (60) à l'entrée de la colonne de benzène (C9). Le paraxylène de haute pureté produit est récupéré en fond de la colonne de purification (C6) et conduit en tant que produit fini par pompage vers le stockage via la ligne (61).

De préférence, la colonne de purification C6 est opérée à basse pression (dans une gamme de pression allant de 1,0 à 2,0 bars absolus au ballon de reflux), de manière à minimiser à la fois la température du rebouilleur et la quantité de chaleur à apporter audit rebouilleur.

Voir la table 1 pour la configuration du rebouilleur et du condenseur de cette colonne selon l'art antérieur.

### Colonne de raffinat (C4)

Le flux de raffinat en provenance de la colonne d'adsorption (P2) est envoyé via la ligne 23 à la colonne de raffinat (C4) qui sépare les C8 aromatiques (raffinat) du désorbant.

Le désorbant récupéré en fond de la colonne (C4) est renvoyé à la section d'adsorption (P2) via la ligne (41).

Le raffinat (coupe C8 aromatiques) est extrait par un soutirage latéral et envoyé via la ligne (40) comme charge de l'unité d'isomérisation des xylènes (P3).

De préférence, la colonne (C4) est opérée à basse pression (dans une gamme de pression allant de 1,0 à 2,0 bars absolus au ballon de reflux) de manière à minimiser à la fois la température du rebouilleur et les calories à apporter audit rebouilleur. Selon l'art antérieur la condensation des vapeurs de tête de la colonne de raffinat (C4) est obtenue en utilisant un aeroréfrigérant.

Voir la table 1 pour la configuration du rebouilleur et du condenseur de cette colonne selon l'art antérieur.

### Colonne de désorbant (non représentée sur les figures 3 ou 4)

Une petite portion du désorbant circulant dans la section d'adsorption (P2) est envoyée à la colonne dite colonne de désorbant de manière à en éliminer les composés lourds qui sinon s'accumuleraient dans la boucle,

Voir la table 1 pour la configuration du rebouilleur et du condenseur de cette colonne selon l'art antérieur.

### Unité d'isomérisation des xylènes (P3)

L'unité d'isomérisation des xylènes (P3) est utilisée pour convertir une charge appauvrie en para -xylène en un flux de xylènes à l'équilibre thermodynamique.

Tout type de catalyseur susceptible d'isomériser les hydrocarbures à 8 atomes de carbones peut être utilisé dans la présente invention. De préférence, on utilise un catalyseur contenant un métal déshydrogénant comme le platine, le palladium ou le nickel et une phase acide, par exemple une alumine dopée, une zéolithe telle que la mordénite, la MFI, la zéolithe Y, ou les tamis moléculaires zéolitiques ou non zéolithiques comportant une acidité tels que les alumino phosphates (AlPO, SAPO). On peut ainsi de manière plus préférée utiliser un catalyseur d'isomérisation comprenant une zéolithe de type structural EUO, telle que la zéolithe EU1, la zéolithe ZSM 50 ou la zéolithe TPZ3 tels que décrites dans les brevets US 4,640,829, EP-B-042226 ou EP- B-051318.

### Dehentaniseur (C7) et stripeur (C8)

L'effluent du réacteur d'isomérisation (section (P3)) est envoyé via la ligne 42 au déheptaniseur (C7) qui sépare l'isomère (composés C8+ aromatiques) d'une coupe légère en C7- récupérée en tête de la dite colonne déheptaniseur (C7). Cette coupe C7- est envoyée via la ligne (71) vers la colonne de stripage (C8) pour séparer les composés légers de la coupe C7. La coupe C7 ainsi stabilisée est récupérée en fond de la colonne de stripage (C8) pour être recyclée via la ligne (81) et combinée avec le distillat en provenance de la colonne (C1) avant d'être envoyée à l'unité de distillation extractive (P1).

Selon l'art antérieur, la condensation partielle des vapeurs de tête de la colonne déheptaniseur (C7) est obtenue par utilisation d'un aéroréfrigérant éventuellement suivi d'un réfrigérant à eau.

Le paramètre limitant qui détermine la pression opératoire du déheptaniseur (C7) est la quantité de composés valorisables (tel que le benzène) qui est perdue à travers le produit dégazé au ballon de reflux. Dans le but de minimiser ces pertes, la pression opératoire au ballon de reflux du déheptaniseur (C7) est généralement maintenue dans une fourchette de pression comprise entre 5,0 et 8,0 bars absolus.

La température résultante au fond du déheptaniseur (C7) est généralement comprise entre 230°C et 250°C, ce qui nécessite l'utilisation d'une source de chaleur de calories à une température supérieure à 250°C. Généralement le fluide calorifique utilisé est soit de la vapeur haute pression, soit un four, ou soit une huile chaude,

La coupe C8+ formée des xylènes et des composés plus lourds récupérée en fond du déheptaniseur (C7) est recyclée via la ligne (72) à l'entrée de la colonne des xylènes (C2). Voir la table 1 pour la configuration du rebouilleur et du condenseur des colonnes (C7) et (C8) selon l'art antérieur.

### Colonne des aromatiques lourds (C3)

Le flux de composés aromatiques en C9+ récupéré en fond de la colonne des xylènes (C2) est envoyé via la ligne (21) à la colonne des aromatiques lourds (C3) qui sépare les composés aromatiques en C9 et C10 des composés plus lourds (tels que le naphtalène) qui ont un effet défavorable sur le catalyseur de transalkylation. La colonne des aromatiques lourds est généralement opérée à basse pression (c'est-à-dire dans une gamme de pression comprise entre 1,0 et 2,0 bars absolus au ballon de reflux) de manière à minimiser à la fois la température et la quantité de chaleur à apporter au rebouilleur de ladite colonne.

Voir la table 1 pour la configuration du rebouilleur et du condenseur de la colonne C3 selon l'art antérieur.

### Unité de transalkylation (P4)

Les composés aromatiques en C9 et C10 récupérés en tête de la colonne des aromatiques lourds (C3) sont envoyés via la ligne (30) pour être mélangés avec le toluène en provenance de la colonne de toluène (C10) pour alimenter l'unité de transalkylation (P4). Cette unité convertit le toluène et les composés aromatiques en C9+ provenant du reformat et de l'isomérat de l'unité (P3) en un mélange de xylènes et de benzène via une réaction limitée par la thermodynamique.

Tout type de catalyseurs de transalkylation est utilisable dans le procédé selon l'invention, par exemple des catalyseurs à base de mordénite ou faujasite décrits dans le brevet US 3,437,710 ou les catalyseurs à base de zéolithes MCM-22 ou béta décrits dans le brevet US 5,030,787, ou les catalyseurs à base de zéolithes mordénite et MFI tels que décrits dans la demande de brevet US2012/0065446. Ces catalyseurs comprennent généralement en sus un composé métallique choisi de préférence parmi le groupe formé par le rhénium, le nickel, le cobalt, le molybdène, le tungstène, le palladium, et le platine.

### Colonne de stabilisation (C11)

L'effluent de l'unité de transalkylation (P4) qui contient du benzène, le toluène non converti, et des aromatiques en C8 et en C9+ est envoyé via la ligne (102) à la colonne de stabilisation (C11) qui sépare les composés plus légers que le benzène, du benzène et des composés aromatiques plus lourds notés C7+.

Le gaz quittant le ballon de reflux de la colonne de stabilisation (C11) est envoyé via la ligne (110) en limite du complexe aromatique.

Une coupe de benzène non purifié est soutirée latéralement et envoyée via la ligne (111) à la colonne de stripage (C8) qui permet de séparer les composés légers de ladite coupe.

Selon l'art antérieur, la condensation partielle des vapeurs de tête de la colonne de stabilisation C11 est obtenue au moyen d'un aéroréfrigérant éventuellement suivi d'un réfrigérant à eau.

Le paramètre limitant qui détermine la pression opératoire de la colonne de stabilisation (C11) est la quantité de composés valorisables (tel que le benzène) qui est perdue à travers le gaz quittant le ballon de reflux.

Dans le but de minimiser ces pertes, la pression opératoire au ballon de reflux de la colonne de stabilisation (C11) est généralement maintenue dans une gamme de pression comprise entre 5,0 et 8,0 bars absolus au ballon de reflux.

La température résultante au niveau du rebouilleur est comprise entre 210°C et 230°C, ce qui nécessite l'utilisation d'une source de calories à apporter au rebouilleur à une température supérieure à 230°C (et qui est généralement constituée soit de vapeur haute pression, soit d'un four ou soit d'une huile chaude).

Voir la table 1 pour la configuration du rebouilleur et du condenseur de cette colonne selon l'art antérieur.

### Colonne de benzène (C9)

A partir de la colonne de benzène (C9), le benzène est extrait en tête en tant que produit final via la ligne (90). La coupe C7+ est extraite en fond de la colonne (C9) et dirigée via la ligne (91) vers la colonne de toluène (C10).

La colonne (C9) est préférentiellement opérée à basse pression dans une gamme allant de 1,0 à 2,0 bars absolus au ballon de reflux de manière à minimiser à la fois la température et la quantité de chaleur à apporter au rebouilleur,

Voir la table 1 pour la configuration du rebouilleur et du condenseur de cette colonne selon l'art antérieur.

### Colonne de toluène (C10)

Le toluène récupéré en tête de la colonne de toluène (C10) est dirigé via la ligne (100) comme charge de l'unité de transalkylation, La coupe C8+ extraite en fond de la colonne (C10) est recyclée via la ligne (101) à la colonne des xylènes (C2) qui sépare les C9+ et plus lourds de la coupe C8 aromatiques alimentant le complexe aromatiques.

Selon l'art antérieur, une fraction de la chaleur de condensation des vapeurs de tête de la colonne de toluène (C10) est utilisée pour apporter les calories nécessaires au rebouilleur de la colonne de benzène (C9).

Dans ce but, la pression opératoire du plateau de tête de la colonne de toluène (C10) est généralement comprise entre 5,0 et 7,0 bars absolus, ce qui permet une température de condensation pour les vapeurs de tête de la colonne de toluène (C10) suffisante pour être utilisée pour le rebouillage de la colonne de benzène (C9).

La température correspondante en fond de la colonne de toluène (C10) se situe entre environ 240°C et 250°C, ce qui nécessite une source de chaleur de calories à apporter au rebouilleur à une température supérieure à 250°C (qui est généralement constituée soit de vapeur haute pression, soit d'un four ou soit d'une huile chaude).

Voir la table 1 pour la configuration du rebouilleur et du condenseur de cette colonne selon l'art antérieur.

La Table 1 selon l'art antérieur, résume les moyens utilisés pour la condensation des vapeurs de tête et le rebouillage du fond des colonnes du complexe aromatiques.

La Table 2 selon la présente invention, permet de distinguer :
- L'ensemble E1 des colonnes qui sont utilisées pour la génération de vapeur basse pression (dans l'exemple selon l'invention les colonnes C3, C4, C5), selon le principe de la figure 2.
- L'ensemble E2 des colonnes qui utilisent la vapeur générée pour l'apport de calories au rebouilleur (dans l'exemple selon l'invention les colonnes C7, C8, C9, C10, C11). Dans cet ensemble certaines colonnes fonctionnent à une pression opératoire plus faible : les colonnes (dans l'exemple selon l'invention les colonnes C7, C8, C10, C11) et certaines (dans l'exemple selon l'invention les colonnes C7 et C11), nécessitent de ce fait une étape de recompression de leur gaz de tête selon le schéma de principe de la figure 1 b.
- Certaines colonnes de l'ensemble E2 (dans l'exemple selon l'invention les colonnes C7, C10, C11), utilisent de la vapeur MP obtenue par 2 étages de compression de la vapeur BP comme fluide caloporteur au rebouillage, selon le schéma de principe de la figure 2 (circuit du flux f2).

La table 3 ci dessous résume les gains énergétiques du complexe aromatique selon l'invention à partir d'une base 1 pour le procédé selon l'art antérieur. Il y a un gain tout à fait significatif sur les 3 postes de consommation de combustible, production de vapeur et d'électricité,

**Table 1**

| **Configuration des rebouilleurs et condenseurs des colonnes selon l'art antérieur,** | | | |
|---|---|---|---|
| | ***Servies*** | ***Moyen de condensation vapeurs de tête*** | ***Moyen de reboullage*** |
| | **Fractionement des Xylenes** | | |
| **C1** | Splitteur de Reformat | Aéroréfrigérant | Vapeur de tête de colonne des Xylenes |
| **C2** | Colonne des Xylenes | Fract. Xylenes - Rebouilleur Splitteur de Reformat Adorption du PX - Rebouilleur Colonne Extrait Adorption du PX - Rebouilleur Colonne Raffinat Distil. Extractive coupe BT - Rebouilleur Strippeur | Four de combustion |

| | **Adsorption du Paraxylene** | | |
|---|---|---|---|
| **C5** | Colonne d'Extrait | Aéroréfrigérant | Vapeur de tête de colonne des Xylenes |
| **C4** | Colonne de Raffinat | Aéroréfrigérant + Réfrigérant à eau | Vapeur de tête de colonne des Xylenes Circuit huile chaude fond de colonne des Xylenes |
| **C6** | Colonne de Purification | Aéroréfrigérant | Charge de la section Adsorption du PX Desordant de la section Adsorption du PX |
| | Colonne du Desorbant | (Pas applicable) | Circuit huile chaude fond de colonne des Xylenes |

| | **Isomerisation des Xylenes** | | |
|---|---|---|---|
| **C7** | Deheptaniseur | Charge du Déhéptaniseur Aéroréfrigérant | Four de combustion |
| **C8** | Strippeur | (pas applicable) | Rebouilleur à vapeur MP, |

| | **Transalkylation** | | |
|---|---|---|---|
| **C3** | Colonnes des Aromatiques Lourds | Aéroréfrigérant | Four de combustion |
| **C11** | Colonne de stabilisation | Aéroréfrigérant | Circuit huile chaude fond de colonne des Xylenes |

| | **Fractionnement Benzène/Toluène** | | |
|---|---|---|---|
| **C9** | Colonne de Benzène | Aéroréfrigérant | Vapeur de tête de la colonne de Toluène |
| **C10** | Colonne de Toluène | Rebouilleur Colonne Benzène Aéroréfrigérant | Four de combustion |

**Table 2**

| **Application à un complexe aromatique selon l'invention** | | | | | |
|---|---|---|---|---|---|
| ***Usités*** | ***Service*** | ***Réduction de la pression opératoire*** | ***Vapeurs de tête utilisées pour génération de vapeur*** | ***Utilisation de la vapeur produite à l'intérieur du Complexe*** | ***Nombtre d'étage de compression de vapeur requis*** |
| | **Fractionement des Xylenes** | | | | |
| **C1** | Splitteur de Reformat | Non | Non | Non | (n.a.) |
| **C2** | Colonne des Xylenes | Non | Non | Non | (n.a.) |

| | **Adsorptiort du Paraxylene** | | | | |
|---|---|---|---|---|---|
| **C5** | Colonne d'Extrait | Non | Oui | Non | (n.a.) |
| **C4** | Colonne de Raffinat | Non | Oui | Non | (n.a.) |
| **C6** | Colonne de Purification | Non | Non | Non | (n.a.) |
| | | | | | |

| | **Isomerisation des Xylenes** | | | | |
|---|---|---|---|---|---|
| **C7** | Déhéptaniseur | Oui | Non | Oui | 2 |
| **C8** | Stripper | Non | (n.a.) | Oui | 0 |

| | **Transalkylation** | | | | |
|---|---|---|---|---|---|
| **C3** | Colonnes des Aromatiques Lourds | Non | Oui | Non | (n.a.) |
| **C11** | Colonne de stabilisation | Oui | Non | Oui | 2 |

| | **Fractionnement Benzène/Toluène** | | | | |
|---|---|---|---|---|---|
| **C9** | Colonne de Benzène | Non | Non | Oui | 1 |
| **C10** | Colonne de Toluène | Oui | Non | Oui | 2 |

| | | | | | |
|---|---|---|---|---|---|
| Note (n.a.) non applicable note 1 : (n.a.) signifie non applicable note 2 : la pression de la colonne C8 est baissée, car cette pression est ajustée à la pression de la colonne C7. | | | | | |

**Table 3**

| | Art antérieur (Fig 3) | Invention (Fig 4) |
|---|---|---|
| Consommation de combustible | 1,0 | 0,71 |
| Consommation de vapeur | 1,0 | 0,84 |
| Consommation électrique | 1,0 | 0,58 |

## Revendications

1. Méthode de récupération de chaleur à basse température, c'est-à-dire comprise entre 100°C et 180 °C, à partir de source de chaleur dite basse température dans un complexe aromatique, comportant au moins une unité de distillation extractive (P1), une unité d'adsorption du paraxylène (P2), une unité d'isomérisation des xylènes (P3) et une unité de transalkylation (P4), les effluents desdites unités étant séparés dans des colonnes à distiller notées de C1 à C11 avec la signification suivante :
C1 colonne de reformat, C2 colonne des xylènes, C3 colonne des aromatiques lourds, C4 colonne de raffinat, C5 colonne d'extrait, C6 colonne de purification, C7 deheptaniseur, C8 stripeur, C9 colonne de benzène, C10 colonne de toluène, C11 colonne de stabilisation,
la dite méthode consistant en la suite d'étapes suivantes :
- 1) on génère de la vapeur basse pression, c'est-à-dire à une pression comprise entre 1 bar abs et 7 bar abs, par échange avec au moins une source de chaleur à basse température, choisie parmi les condenseurs de certaines colonnes à distiller du complexe formant un premier ensemble E1, constitué des colonnes C3, C4 et C5,
- 2) on augmente la pression de la vapeur générée à l'issue de l'étape 1 dans au moins une étape de compression, pour créer de la vapeur MP,
- 3) on modifie les conditions opératoires du procédé par une diminution de la pression opératoire d'une ou plusieurs colonnes de distillation du complexe, de manière à abaisser la température de leur rebouilleur au dessous de la température de la vapeur issue de l'étape 2,
- 4) on utilise au moins une partie de la vapeur BP issue de l'étape 1 comme fluide caloporteur apporté au rebouilleur de la colonne C9, et au moins une partie de la vapeur MP issue de l'étape 2 après recompression, comme fluide caloporteur apporté aux rebouilleurs des colonnes C7, C8, C10 et C11, l'ensemble des colonnes C7, C8, C9, C10 et C1 1 formant l'ensemble E2 distinct de E1,

2. Méthode de récupération de chaleur selon la revendication 1, dans laquelle on utilise une partie de la vapeur basse pression issue de l'étape 1 pour générer de l'électricité.

3. Méthode de récupération de chaleur selon la revendication 1, dans laquelle l'étape 2 est réalisée au moyen d'un compresseur à deux étages.

4. Méthode de récupération de chaleur selon la revendication 1, dans laquelle l'étape 2 est réalisée au moyen d'un éjecteur utilisant de la vapeur HP comme fluide moteur.

5. Méthode de récupération de chaleur selon la revendication 1, dans laquelle la vapeur basse pression (BP) issue de l'étape 1, ou la vapeur moyenne pression (MP) issue de l'étape 2 est utilisée comme fluide caloporteur pour apporter de la chaleur à l'air de combustion des fours dans des préchauffeurs d'air.

## Patentansprüche

1. Wärmerückgewinnungsverfahren mit Niedertemperatur, das heißt zwischen 100 °C und 180 °C, ausgehend von einer sogenannten Niedertemperaturwärmequelle in einem Aromatenkomplex, umfassend mindestens eine Extraktivdestillationseinheit (P1), eine Adsorptionseinheit des Paraxylols (P2), eine Isomerisierungseinheit der Xylole (P3) und eine Transalkylierungseinheit (P4), wobei die Abströme der Einheiten in Destillationskolonnen getrennt werden, die mit C1 bis C11 bezeichnet sind, die die folgende Bedeutung haben:
C1 Kolonne des Reformats, C2 Kolonne der Xylole, C3 Kolonne der schweren Aromaten, C4 Raffinatkolonne, C5 Extraktkolonne, C6 Reinigungskolonne, C7 Deheptanizer, C8 Stripper, C9 Benzolkolonne, C10 Toluolkolonne, C11 Stabilisierungskolonne,
wobei das Verfahren in der Abfolge der folgenden Schritte besteht:
- 1) Erzeugen von Niederdruckdampf, das heißt mit einem Druck, der zwischen 1 bar abs und 7 bar abs beträgt, durch Austausch mit mindestens einer Niedertemperaturwärmequelle, die ausgewählt wird aus den Kondensatoren von bestimmten Destillier-Kolonnen des Komplexes, die eine erste Anordnung E1 bilden, die aus den Kolonnen C3, C4 und C5 besteht,
- 2) Erhöhen des Drucks des Dampfes, der am Ende des Schrittes 1 generiert wird, in mindestens einem Kompressionsschritt, um Mitteldruckdampf zu erzeugen,
- 3) Ändern der Betriebsbedingungen des Verfahrens durch ein Verringern des Betriebsdrucks von einer oder mehreren Destillationskolonnen des Komplexes derart, um die Temperatur ihres Reboilers unter die Temperatur des Dampfs zu senken, der aus dem Schritt 2 hervorgegangen ist,
- 4) Verwenden von mindestens einem Teil des Niederdruckdampfes, der aus dem Schritt 1 hervorgegangen ist, als Wärmeträgerfluid, das dem Reboiler der Kolonne C9 zugeführt wird, und von mindestens einem Teil des Mitteldruckdampfes, der aus Schritt 2 hervorgegangen ist, nach Rekomprimieren als Wärmeträgerfluid, das den Reboilern der Kolonnen C7, C8, C10 und C11 zugeführt wird, wobei die Anordnung der Kolonnen C7, C8, C9, C10 und C11 die Anordnung E2 bilden, die von E1 verschieden ist.

2. Wärmerückgewinnungsverfahren nach Anspruch 1, wobei ein Teil des Niederdruckdampfes verwendet wird, der aus Schritt 1 hervorgegangen ist, um Strom zu erzeugen.

3. Wärmerückgewinnungsverfahren nach Anspruch 1, wobei der Schritt 2 mittels eines zweistufigen Verdichters durchgeführt wird.

4. Wärmerückgewinnungsverfahren nach Anspruch 1, wobei der Schritt 2 mittels eines Ejektors durchgeführt wird, der Hochdruckdampf als Antriebsfluid verwendet.

5. Wärmerückgewinnungsverfahren nach Anspruch 1, wobei der Niederdruckdampf (BP), der aus Schritt 1 hervorgegangen ist, oder der Mitteldruckdampf (MP), der aus Schritt 2 hervorgegangen ist, als Wärmeträgerfluid verwendet wird, um derVerbrennungsluft der Öfen in Luftvorwärmern Wärme zuzuführen.

## Claims

1. A method of recovering low-temperature heat, that is to say between 100°C and 180°C, from a so-called low-temperature heat source in an aromatic complex comprising at least one extractive distillation unit (P1), a paraxylene adsorption unit (P2), a xylenes isomerisation unit (P3) and a transalkylation unit (P4), the effluents from said units being separated in distillation columns identified as C1 to C11, bearing the following meanings:
C1 reformate column, C2 xylenes column, C3 heavy aromatics column, C4 raffinate column, C5 extract column, C6 purification column, C7 deheptaniser, C8 stripper, C9 benzene column, C10 toluene column and C11 stabilisation column,
said method consisting of the succession of the following steps:
- 1) generating low-pressure steam, that is to say at a pressure of between 1 bar absolute and 7 bars absolute, by exchange with at least one low-temperature heat source selected from the condensers of certain distillation columns of the complex forming a first assembly E1, formed by columns C3, C4 and C5,
- 2) increasing the pressure of the steam generated at the issue from step 1 in at least one compression step to create MP steam,
- 3) modifying the operating conditions of the process by a reduction in the operating pressure of one or more distillation columns of the complex so as to reduce the temperature of their reboiler below the temperature of the steam issuing from step 2, and
- 4) using at a least a part of LP steam issuing from step 1 as a heat transfer fluid supplied to the reboiler of the column C9 and at least a part of the MP steam issuing from step 2 after recompression as a heat transfer fluid supplied to the reboilers of the columns C7, C8, C10 and C11, the assembly of the columns C7, C8, C9, C10 and C11 forming the assembly E2 which is separate from E1.

2. A heat recovery method according to claim 1 wherein a part of the low-pressure steam issuing from step 1 is used to generate electricity.

3. A heat recovery method according to claim 1 wherein step 2 is carried out by means of a two-stage compressor.

4. A heat recovery method according to claim 1 wherein step 2 is performed by means of an ejector using HP steam as a motive fluid.

5. A heat recovery method according to claim 1 wherein the low-pressure (LP) steam issuing from step 1 or the medium-pressure (MP) steam issuing from step 2 is used as a heat transfer fluid to supply heat to the combustion air of the furnaces in air preheaters.
